# EUROPEAN PATENT APPLICATION

(11) **EP 4 748 338 A1**
(43) Date of publication of application: **27.05.2026**
(21) Application number: 25217496.6
(22) Date of filing: 20.11.2025
(51) Int. Cl.: A61B 34/30

(54) **VISUALIZATION OF EFFECTS OF DEVICE MOVEMENTS IN AN OPERATING ROOM**

(30) Priority: 20.11.2024 US 202418954178
(71) Applicant: Cilag GmbH International, 6300 Zug (CH)
(72) Inventor: SHELTON IV,, Frederick E., Cincinnati, 45242 (US); BRADY,, John E., Cincinnati, 45242 (US); JONES,, Shannon L., Cincinnati, 45242 (US); HARRIS,, Jason L., Cincinnati, 45242 (US); ARONHALT,, Jacqueline Corrigan, Cincinnati, 45242 (US); JAYME,, Madeleine, Cincinnati, 45242 (US); KAMINSKI,, Benjamin M., Cincinnati, 45242 (US); DECK,, Andrew C., Cincinnati, 45242 (US); MUELLER,, Karl W., Cincinnati, 45242 (US); ACKERMANN, III,, Richard Joseph, Cincinnati, 45242 (US); DRAGINOFF,, Carl Joseph, Cincinnati, 45242 (US); ZECKEL RIVARD,, Monica Louise, Cincinnati, 45242 (US); COWPERTHWAIT,, Matthew David, Cincinnati, 45242 (US); MESSERLY,, Jeffrey David, Cincinnati, 45242 (US); LEIMBACH,, Richard L., Cincinnati, 45242 (US); ZEINER,, Mark, Cincinnati, 45242 (US); SCULLY,, Sarah A., Cincinnati, 45242 (US)
(74) Representative: Carpmaels & Ransford LLP

(57) **Abstract**

Devices and method for visualizing effects of device movements in an operating room. The device may identify candidate motions of a first robotic arm configured to place a first end effector in a target end effector position internal to a patient. The device may determine, for a first candidate motion, a first number of associated interactions in which the first robotic arm and a second robotic arm will co-occupy space external to the patient. The device may determine, for a second candidate motion, a second number of associated interactions in which the first robotic arm and the second robotic arm will co-occupy space external to the patient. The device may select the first candidate motion or the second candidate motion based on the first and second number of interactions. The device may generate a control signal based on the selected candidate motion of the first robotic arm.

## Description

### Cross-Reference to Related Applications

This application claims the benefit of the following, the disclosures of which are incorporated herein by reference in its entirety:
- Provisional U.S. Patent Application No. 63/602,040, filed November 22, 2023,
- Provisional U.S. Patent Application No. 63/602,028, filed November 22, 2023,
- Provisional U.S. Patent Application No. 63/601,998, filed November 22, 2023,
- Provisional U.S. Patent Application No. 63/602,003, filed November 22, 2023,
- Provisional U.S. Patent Application No. 63/602,006, filed November 22, 2023,
- Provisional U.S. Patent Application No. 63/602,011, filed November 22, 2023,
- Provisional U.S. Patent Application No. 63/602,013, filed November 22, 2023,
- Provisional U.S. Patent Application No. 63/602,037, filed November 22, 2023,
- Provisional U.S. Patent Application No. 63/602,007, filed November 22, 2023,
- Provisional U.S. Patent Application No. 63/603,031, filed November 27, 2023, and
- Provisional U.S. Patent Application No. 63/603,033, filed November 27, 2023.

This application is related to the following, filed contemporaneously, the contents of each of which are incorporated by reference herein:
- U.S. Patent Application No. 18/954,186, filed November 20, 2024, entitled METHOD FOR MULTI-SYSTEM INTERACTION,
- U.S. Patent Application No. 18/954,195, filed November 20, 2024, entitled, VISUALIZATION OF AN INTERNAL PROCESS OF AN AUTOMATED OPERATION,
- U.S. Patent Application No. 18/954,199, filed November 20, 2024, entitled VISUALIZATION OF AUTOMATED SURGICAL SYSTEM DECISIONS,
- U.S. Patent Application No. 18/954,205, filed November 20, 2024, entitled VISUALIZATION OF EFFECTS OF DEVICE PLACEMENT IN AN OPERATING ROOM, and
- U.S. Patent Application No. 18/954,214, filed November 20, 2024, entitled DISPLAY OF COMPLEX AND CONFLICTING INTERRELATED DATA STREAMS.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

Devices and methods for visualizing effects of device placement in an operating room. An example device may include a processor configured to perform one or more actions. The device may receive an indication of a plurality of steps of a surgical procedure associated with a patient. One or more steps in the plurality of steps of the surgical procedure may involve use of a first robotic arm having a first end effector attached and a second robotic arm. The device may identify a first candidate motion and a second candidate motion of the first robotic arm configured to place the first end effector in a target end effector position internal to the patient. The device may determine, for the first candidate motion, a first number of associated interactions in which the first robotic arm and the second robot arm co-occupy space external to the patient during the surgical procedure. The device may determine, for the second candidate motion, a second number of associated interactions in which the first robotic arm and the second robot arm co-occupy space external to the patient during the surgical procedure. The device may select a candidate motion of the first robotic arm, from the first candidate motion and the second candidate motion, based on the first number of interactions and the second number of interactions. The device may generate a control signal based on the selected candidate motion of the first robotic arm.

The device may determine, during a first step in the plurality of surgical procedure steps, a current arm position of the first robotic arm and a current arm position of the second robotic arm that are external to a patient. The device may determine, during a second step in the plurality of surgical procedure steps, the target end effector position of the first end effector, wherein the first candidate motion and a second candidate motion of the first robotic arm are identified based on the current arm positions of the first and second robotic arms and the plurality of steps of the surgical procedure.

The control signal may be configured to indicate the selected candidate motion of the first robotic arm. The control signal may be configured to indicate one or more of: the first candidate motion, the first number of interactions, the second candidate motion, the second number of interactions, a recommendation to move the first robotic arm according to the selected candidate motion, an order in which to perform the selected candidate motion and a motion of the second robotic arm, or a time at which to perform the selected candidate motion.

Each step in the plurality of steps of the surgical procedure may be associated with a surgical site internal to the patient, a second end effector is attached to a distal end of the second robotic arm. The device may identify a set of candidate motions, comprising the first candidate motion and the second candidate motion, based on the plurality of steps of the surgical procedure. Each candidate motion in the set of candidate motions may allow the first end effector and the second end effector to access the surgical site at a given step in the plurality of steps of the surgical procedure.

On a condition that the first number of interactions is less than the second number of interactions, the device may select the first candidate motion. On a condition that the first number of interactions is greater than the second number of interactions, the device may select the second candidate motion.

The target end effector position of the first end effector may be a first position. The device may determine an updated current arm position of the first robotic arm, external to the patient, based on the first robotic arm moving according to the selected candidate motion. The device may determine a second target end effector position of the second end effector, during a third step in the plurality of surgical procedure steps. The second target end effector position may be internal to the patient.

The device may determine, based on the updated current arm position of the first robotic arm, the current arm position of the second robotic arm, and the plurality of steps of the surgical procedure, a third candidate motion of the second robotic arm that will place the second end effector in the second target end effector position. The third candidate motion of the second robotic arm may be associated with a third number of interactions in which the first robotic arm and the second robot arm will co-occupy space during the surgical procedure;

The device may determine, based on the updated current arm position of the first robotic arm, the current arm position of the second robotic arm, and the plurality of steps of the surgical procedure, a fourth candidate motion of the second robotic arm that will place the second end effector in the second target end effector position. The fourth candidate motion of the second robotic arm may be associated with a fourth number of interactions in which the first robotic arm and the second robot arm will co-occupy space during the surgical procedure. The device may select a candidate motion of the second robotic arm, from the third candidate motion and the fourth candidate motion, based on the third number of interactions and the fourth number of interactions. The device may generate a control signal based on the selected candidate motion of the second robotic arm.

One or more steps in the plurality of steps of the surgical procedure may involve use of a third robotic arm. The device may predict an effect, caused by the selected candidate motion of the first robotic arm, on a future motion of a third robotic arm, wherein the control signal is further configured to indicate the effect.

The device may receive user preference information and a patient position associated with the surgical procedure. The device may determine a surgical constraint based on at least one of the user preference information or the patient position. The device may select the candidate motion of the first robotic arm, from the first candidate motion and the second candidate motion, based on the surgical constraint.

The first robot arm may include a plurality of joints configured to move the first robot arm. The device may select, from the plurality of joints, a joint of the first robotic arm to articulate to achieve the selected candidate motion.

### BRIEF DESCRIPTION OF THE DRAWINGS

FIG. 1 is a block diagram of a computer-implemented surgical system.
FIG. 2 illustrates an example surgical system in a surgical operating room.
FIG. 3 illustrates an example surgical hub paired with various systems.
FIG. 4 illustrates an example situationally aware surgical system.
FIG. 5 illustrates example joint movements of a robotic arm.
FIG. 6 illustrates an example operating room arrangement of multiple robotic arms.
FIG. 7 illustrates an example display to a user of areas of potential interactions between surgical devices.
FIG. 8 illustrates an example display to a user of potential interactions between robotic arms during a surgical procedure.
FIG. 9 illustrates an example display to a user of zones of robotic arm movements.
FIG. 10 illustrates example robotic arm movements based on the type of procedure being performed and/or a step of the procedure.

### DETAILED DESCRIPTION

A more detailed understanding may be had from the following description, given by way of example in conjunction with the accompanying drawings.

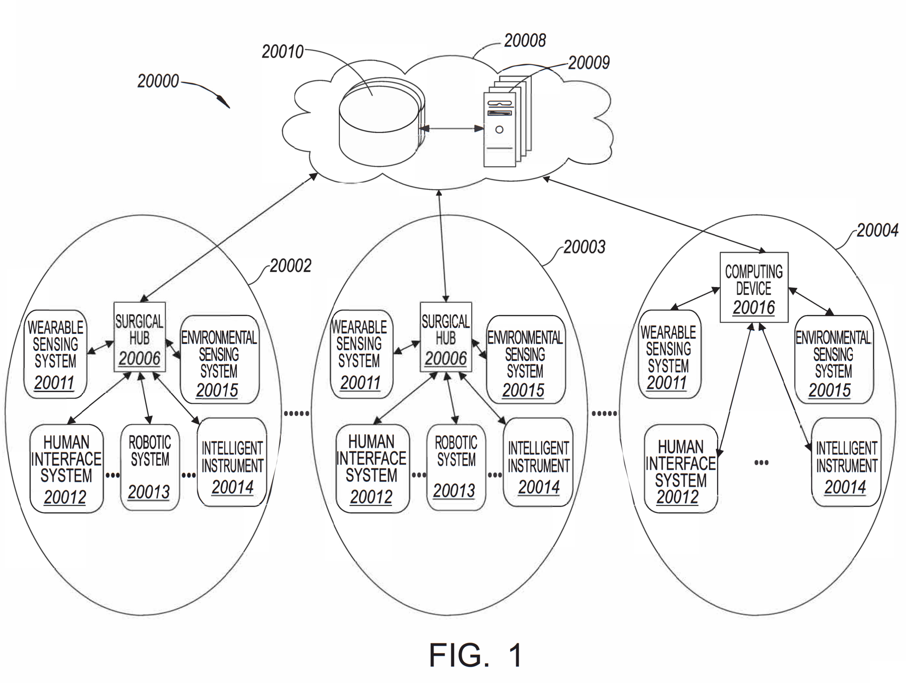

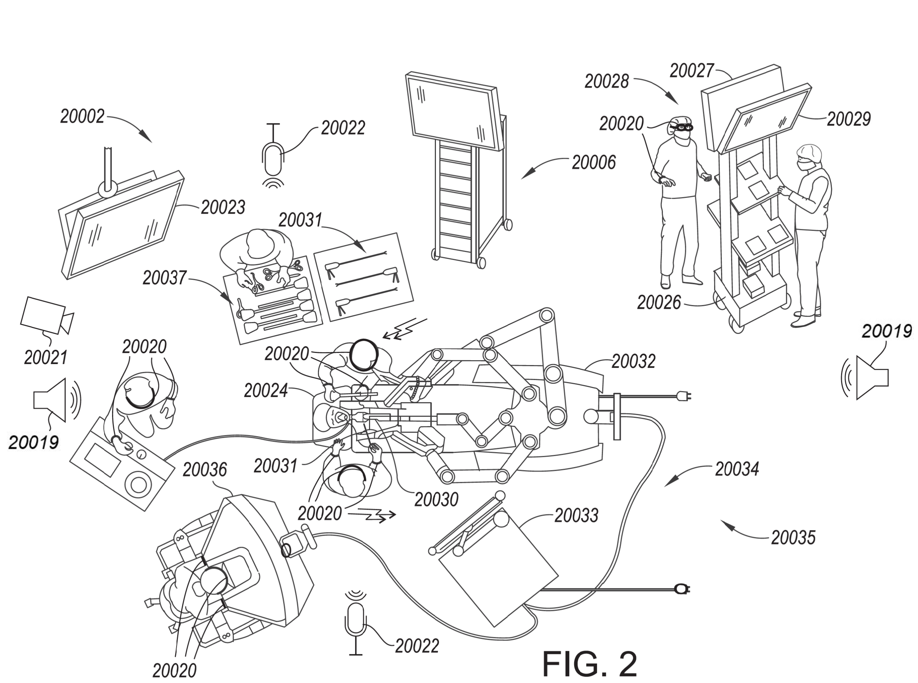

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

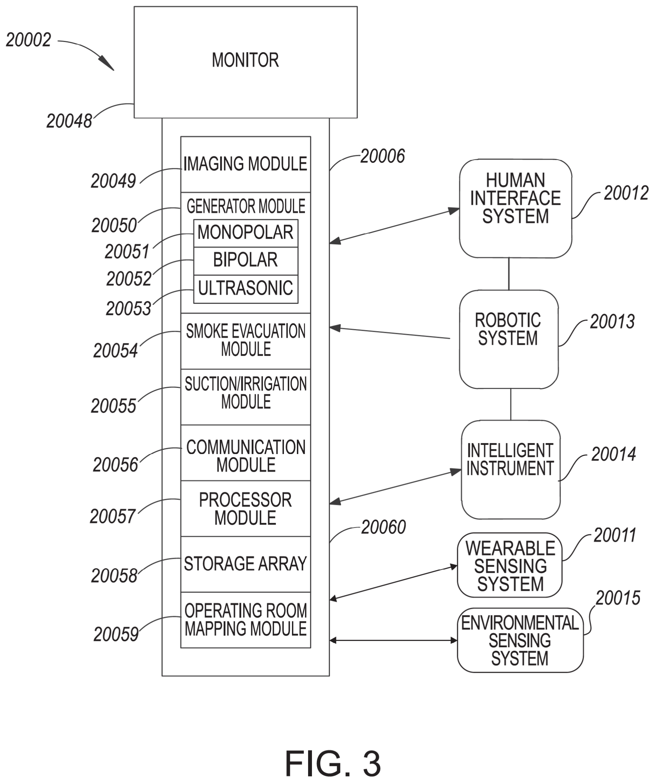

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

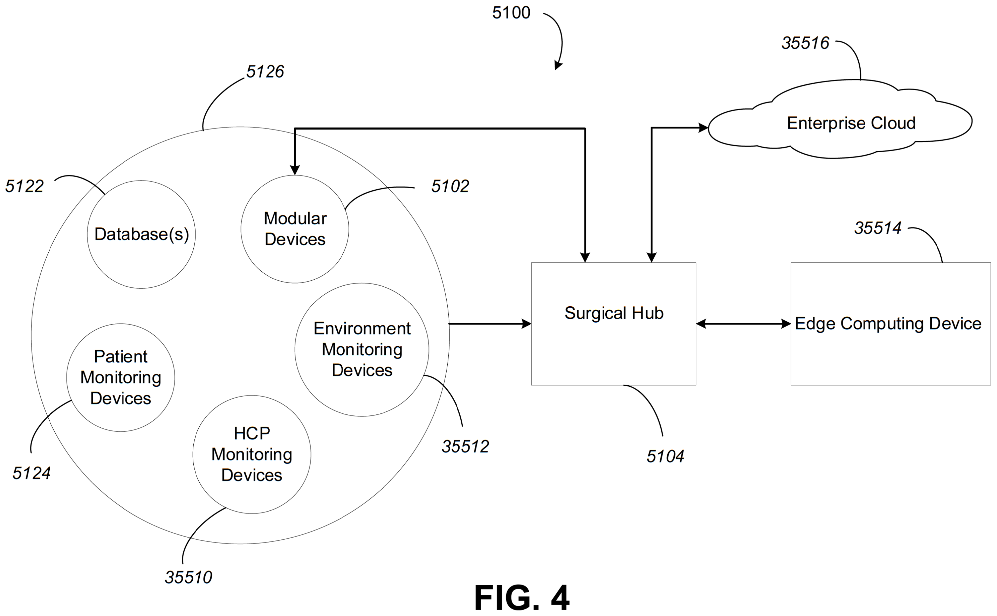

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

Robotic arm movement may be improved (e.g., optimized) to control interactions between arms outside the body. A system may automate decision making for optimizing arm movements.

On a robotic arm, there may be a large number of options (e.g., infinite options) that can result in locating the end effector in the desired location. Robotic arm movements may be governed by the simplest and/or most efficient way to move the end effector to the desired location inside the patient. This may cause the robotic arms to collide with one another during the surgical procedure (e.g., which may limit the surgeon's access) or to become so entangled that the procedure is stopped to allow the OR team to untangle and reposition the robot arms prior to resuming surgery. In a digitally connected OR, additional data feeds (e.g., external cameras in the OR), beyond just the robotic arm placement, may be used to inform the robot of its external arm locations and assist its ability to prevent collision and/or entanglement via informed decisions of what kinematic movements to make outside the patient (e.g., which joints to move, how much to move, etc.).

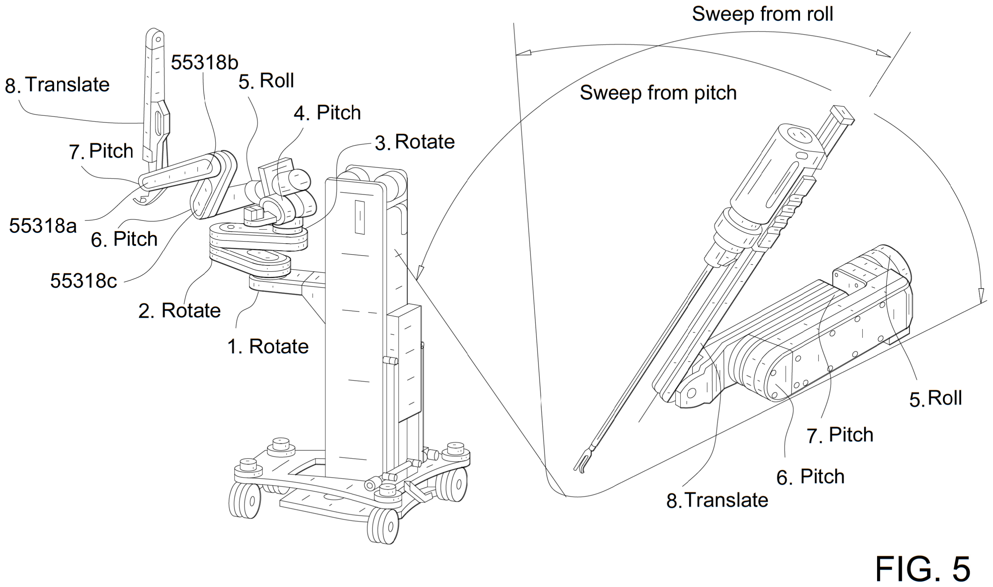

FIG. 5 illustrates example joint movements of a robotic arm attached to a base. As shown, the arm may be able to move in the x, y, and z directions. The joints may control the pitch, roll, and rotation of an end effector attached to the arm. The arm may have a mechanism (e.g., slide) for translation of the end effector. The joints may cause the end effector to make small movements inside the patient (e.g., in the surgical cavity). The arm movements correlated to the small internal movements may be large (e.g., creating a considerable sweep of the arm).

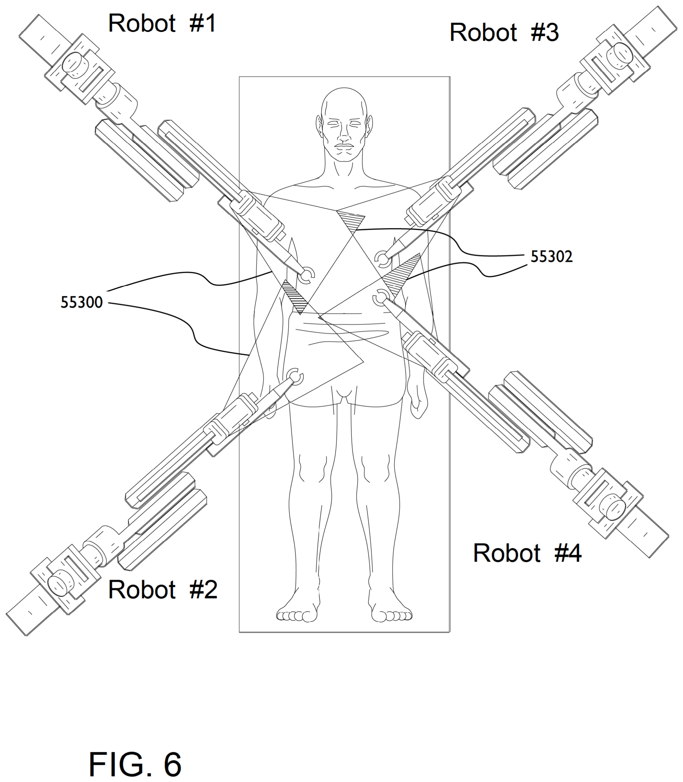

FIG. 6 illustrates an example operating room arrangement of multiple robotic arms. As shown, multiple robotic arms may be present in a relatively small space in an OR. The arms may have ranges of motion that overlap (e.g., as shown at 55300 and 55302). In this case, the arms may collide unless adjustments are made to reduce the potential interactions. For example, one or more of the arms may not be allowed to occupy the overlapping space (e.g., at a given time). As described herein, the system may determine potential robot arm position placement and kinematic forecasting of resulting positions, movements, and/or interactions. The system may determine (and display) options for the HCP to choose from (e.g., and request that the HCP provide input). The system may output a simple, understandable display of context and choices.

Devices and methods for visualizing effects of device placement in an operating room. An example device may include a processor configured to perform one or more actions. The device may receive an indication of a plurality of steps of a surgical procedure associated with a patient. One or more steps in the plurality of steps of the surgical procedure may involve use of a first robotic arm having a first end effector attached and a second robotic arm. The device may identify a first candidate motion and a second candidate motion of the first robotic arm configured to place the first end effector in a target end effector position internal to the patient. The device may determine, for the first candidate motion, a first number of associated interactions in which the first robotic arm and the second robot arm co-occupy space external to the patient during the surgical procedure. The device may determine, for the second candidate motion, a second number of associated interactions in which the first robotic arm and the second robot arm co-occupy space external to the patient during the surgical procedure. The device may select a candidate motion of the first robotic arm, from the first candidate motion and the second candidate motion, based on the first number of interactions and the second number of interactions. The device may generate a control signal based on the selected candidate motion of the first robotic arm.

The system may display choices of powered device joint motion (e.g., based on preferences or concern of where and/or when the devices may interact. The system may monitor the positions and orientation of the portions of the devices outside of the body. The system may monitor the current position of the devices and predicting future positions of the devices based on the surgical tasks or procedure plan. The prediction may be aggregated into options for presentation to the user. The options may indicate interaction location, timing, or magnitude. The system may choose instrument joint motion controls based on the feedback from the user (e.g., what spaces have preferred operational space and/or spaces to keep as clear as possible). The powered devices may be robotic arm assemblies and/or tools. For example, on a condition that the first number of interactions is less than the second number of interactions, the device may select the first candidate motion. On a condition that the first number of interactions is greater than the second number of interactions, the device may select the second candidate motion.

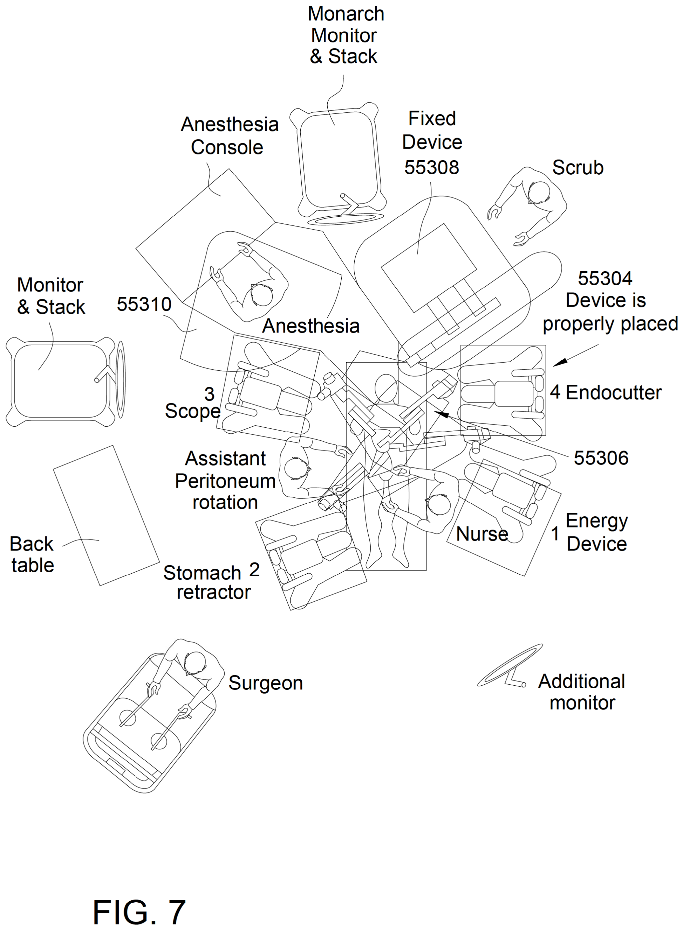

FIG. 7 illustrates an example display to a user of areas of potential interactions between surgical devices. As shown at 55304, the system may indicate that a device is properly placed. The display further shows areas in which multiple robot arms may occupy the same space. For example, as shown at 55306, the arm with endocutter 4 may share an overlapping space with the arm attached to energy device 1. The system may determine sequential movements of the two arms to prevent a collision in the overlapping space. For example, if the endocutter arm is moving through the space to reposition, the energy device arm may remain still. The energy device arm may be moved once the endocutter arm has finished moving and exited the overlapping space.

FIG. 7 further illustrates an example of a fixed (e.g., non-moving) device 55308. The fixed device 55308 may be a ventilator (e.g., a Monarch smart ventilator). The ventilator may be placed (e.g., and fixed) at the patient's head (e.g., because the ventilator must be attached at the patient's mouth and/or nose). FIG. 7 further illustrates an example area 55310 in which an HCP (e.g., the anesthesiologist) will occupy during one or more parts of the surgery. For example, throughout a surgery, the anesthesiologist may need to have access to the patient's head to properly check and maintain sedation.

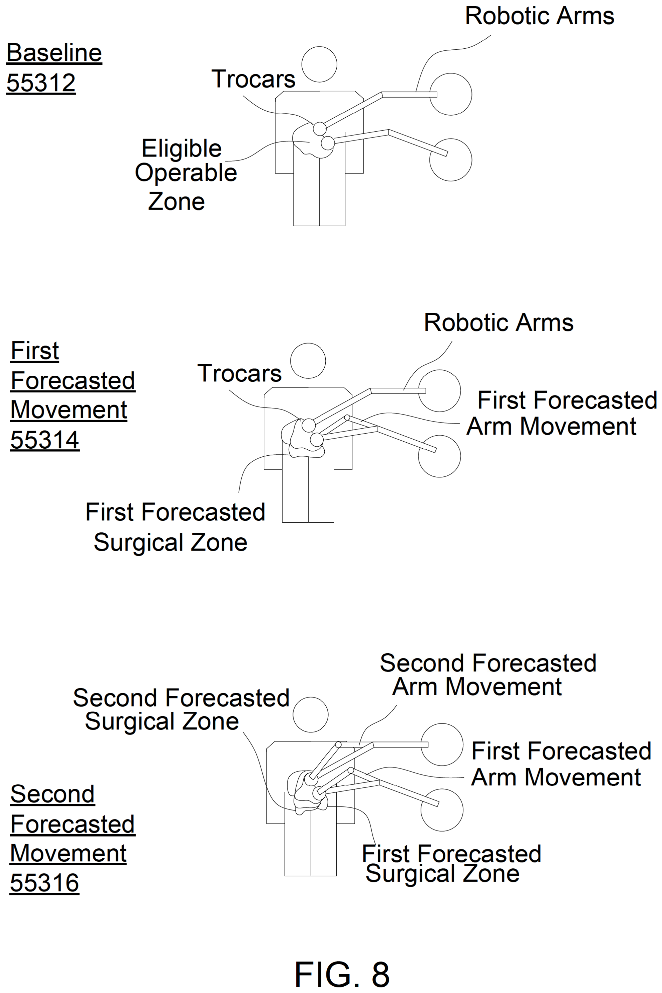

FIG. 8 illustrates an example display to a user of potential interactions between robotic arms during a surgical procedure. In a baseline position 55312 (shown in the top figure), the two robotic arms may be in an initial location. This initial location may be at a time just after the instruments attached to the ends of the arms were inserted into the patient via trocars. Based on this initial position, the system may determine an eligible operable zone. The eligible operable zone may indicate the areas in the patient's body that are accessible to the end effectors without moving arm joints external to the patient's body.

The target end effector position of the first end effector may be a first position. The device may determine an updated current arm position of the first robotic arm, external to the patient, based on the first robotic arm moving according to the selected candidate motion. The device may determine a second target end effector position of the second end effector, during a third step in the plurality of surgical procedure steps. The second target end effector position may be internal to the patient.

The device may determine, based on the updated current arm position of the first robotic arm, the current arm position of the second robotic arm, and the plurality of steps of the surgical procedure, a third candidate motion of the second robotic arm that will place the second end effector in the second target end effector position. The third candidate motion of the second robotic arm may be associated with a third number of interactions in which the first robotic arm and the second robot arm will co-occupy space during the surgical procedure.

The device may determine, based on the updated current arm position of the first robotic arm, the current arm position of the second robotic arm, and the plurality of steps of the surgical procedure, a fourth candidate motion of the second robotic arm that will place the second end effector in the second target end effector position. The fourth candidate motion of the second robotic arm may be associated with a fourth number of interactions in which the first robotic arm and the second robot arm will co-occupy space during the surgical procedure. The device may select a candidate motion of the second robotic arm, from the third candidate motion and the fourth candidate motion, based on the third number of interactions and the fourth number of interactions. The device may generate a control signal based on the selected candidate motion of the second robotic arm.

The surgeon may determine to move one of the arms using joints external to the patient (e.g., to access an area not in the eligible operable zone). The first forecasted move 55314 (shown in the middle figure) illustrates an example movement of one of the arms. As shown, the system may update the displayed image to show an updated operable zone (e.g., a first forecasted surgical zone). The system may keep a depiction of the initial positioning of the arm. This may allow the surgeon to better visualize what the movement will look like. The surgeon may similarly move the other arm using joints external to the patient. The second forecasted move 55316 (shown in the bottom figure) illustrates an example movement of the arm. As shown, the system may update the displayed image to show an updated operable zone (e.g., a second forecasted surgical zone). The system may keep a depiction of the initial positioning of the arm. These depictions may allow the surgeon to keep track of the positioning of the arms relative to each other, which may help the surgeon avoid collisions.

The system may detect sub-optimal equipment set up. The system may be able to project guidance markings on the OR floor and/or equipment to assist OR staff set up the room.

The system may include one or more OR ceiling or boom-mounted overhead cameras to monitor the presence and location of people and equipment in the OR. Cameras may connect with a computer system running computer vision models (e.g., in real time) capable of detecting the position of various OR equipment and people.

The multiple cameras may be mounted at configured distances (e.g., such that the system may utilize information about their relative position to each other and the floor). For example, the cameras may be used to register the measured positions of objects in the camera image within a virtual multi-dimensional reconstruction of the OR.

Robots may be equipped with means for determining the location of one arm relative to another in three-dimensional space. For example, another may monitor arms and provide relative measurements of one with respect to the other. For example, the system may determine the relative measurements through imaging of the OR. One or more cameras within the OR may be used to generate this information. The cameras may be separate from, or on the robot itself. The system may determine the relative measurements based on magnetic sensors, ultrasonic pinging, etc. This additional data feed may be used to determine the location of the devices relative to each other.

The system may utilize knowledge of surgical context for a given procedure (e.g., surgeon, procedure, surgical tool preference card information, etc.) to determine which pieces of equipment should be in place for the surgery (the "Necessary Components"). For example, the system may determine which robotic system components (e.g., robotic arm bases, a flexible robotic system, etc.) to have in the room. Surgical robot systems from different manufacturers may be present in the room.

Components may be the source of collisions and/or setup issues. Components may create access issues for OR staff if not properly positioned.

Object detection algorithms running on the system may be trained to detect the position/location of components and the OR table. The OR table position may be used as a datum or reference location for placement of other large components to define locations for each piece of equipment.

With knowledge of the OR table position and other objects in the multi-dimensional reconstruction, the system may be able to compare the measured positions of components against a pre-configured database of acceptable setup positions (e.g., "Go" and "No Go" positions). Acceptable setup positions may include the position and orientation of components (e.g., robotic arm base components on the floor). Acceptable setup positions may be defined as a polygon shape on the OR floor (e.g., within which the position is acceptable). Orientation may include a range of angles.

If a piece of OR equipment is determined to be in a "No Go" position, the system may alert the user. For example, the user may be alerted based on the system projecting images on the floor of the OR giving visual display of "Go" zones. Projection may be accomplished via one or more ceiling- or boom-mounted projectors that are registered to the same coordinate system as the cameras. A monitor may show the virtual reconstruction of the OR and indicate equipment that is out of position. With the projector system, if a piece of equipment were out of position, the projector may display colors or patterns on the floor or equipment.

Camera localization and detection of robot arms may be used for (e.g., optimal) placement prediction. Hybrid on-screen and off-screen capital placement (e.g., OR set-up) may be performed using OR spotlights/projectors.

Simplified colored light spotlights (e.g., projectors) in multiple colors may be part of the camera hub, or ceiling mounted. The spotlights may illuminate a specified area with a given color (e.g., red). The on-screen display may indicate to the user to place a specified piece of equipment into the illuminated area (e.g., "place endoscope robotic arm in red area"). This may be performed sequentially (e.g., using the same color), or in parallel (e.g., using multiple spotlights of different colors).

If a spotlight is in line with camera, the camera may provide a feedback loop to the spotlight to adjust the color or intensity to visually provide feedback and indicate if an item is placed correctly (e.g., switch from red to green) or to help the OR team to optimize placement (e.g., increase light intensity as position is optimized).

Color selection, commands, and feedback may depend on whether the OR team arranges the OR in series or in parallel.

The system may select robot arm movements (e.g., optimal movements to prevent robot arm entanglement). If a robotic arm interaction is anticipated, the system may perform one or more actions.

This algorithm would be on a processor located somewhere in the OR. The algorithm may subscribe to information from the cameras and/or pre-planning information. From knowledge about the placements/positions of items in the room and appropriate mathematical calculations, the software may predict collisions. The system may recommend changes to arm positions and alert the surgeon (e.g., in real-time).

The algorithm may recommend robotic arm adjustments during surgery. Setting adjustment to one or more robots may be performed to enable continued function on a 'primary' arm. Settings may include restricted motion/speed, joint adjustment, yielding to prioritized tasks.

The system may detect an expected collision of robotic arm components outside of the body. The system may include a follower robotic arm and a commanded/active robotic arm. The commanded robotic arm may be (e.g., actively) controlled by the surgeon and may collide with the follower arm outside of the body (e.g., as some end effector positions may cause robotic arm configurations with a large degree of movement, potentially infringing on collision zones or the physical envelope of another arm).

As the commanded arm moves, a system may monitor its arm position. If a collision is anticipated based on the arm position outside of the body between the commanded arm and a follower arm, the follower arm may maintain its end effector position using the degrees of freedom in its wrist (e.g., end effector roll, articulation, etc.). The follower arm may reconfigure its joints out of the body to move the arm linkages out of the way of the actively commanded arm. The surgeon may be notified that the follower arm is in motion due to a collision avoidance maneuver. The movement of the follower arm may have limits. If the collision cannot be avoided by dynamic reconfiguration, the surgeon may be notified that the maneuver is out of range and will not be attempted.

Dynamic reconfiguration maneuvers may be used to avoid robotic arm collisions.

Reconfiguration may involve speed scaling. As a component of a robotic system nears or enters the collision space of another system or obstacle, the controlled speed of the robotic system may be decreased (e.g., dynamically) based on distance from the hazard or potentially colliding object. The slowing of the robotic system may increase as proximity to the colliding object increases (e.g., up to the extent that the robot may cease motion entirely in the direction towards the obstacle). This speed scaling function may be applied to motion in the direction of the obstacle (e.g., exclusively). The speed scaling may prevent collision while maintaining normal operation in safe zones/directions or allow for retreat from the obstacle.

Reconfiguration may involve inertially-weighted motion scaling. The collision space of the obstacle may increase in proportion to the calculated inertia of the robotic arm (e.g., based on current moving speed and payload/weight of arm and end effector tooling). This may create additional reaction and slowing time when the robotic system is less capable of ceasing motion due to inertial loads.

Reconfiguration may involve arrest control. In some cases (e.g., where a collision is imminent or will create a hazard), motion of the robot may be locked out (e.g., entirely) of a specific collision zone (e.g., defined as a radius from an object) to prevent the hazardous situation from occurring. If inertial loads are too great, dynamic braking (e.g., if available) may be (e.g., automatically) implemented to prevent the collision.

The system may detect that a first robot (e.g., Robot A) is expected to collide with a second robot (e.g., Robot B). Robot A may not be fed information or controlled by the global controller that runs the OR vision systems and Robot B. In this case, Robot B may yield to Robot A. The system may stop motion and/or warn the surgeon to move or reconfigure the end effector or arm body. If an arm-based out-of-body collision is expected and Robot A is the moving robot, Robot B may reconfigure (e.g., without moving the end effector) to get as far out of the way as possible.

If Robot B is moving and Robot A is not controlled by the OR system, Robot B may establish zones within which it cannot operate or is slowed due to the presence of Robot A. The slowing or keep out zones may prevent Robot B from contacting Robot A.

The control system may prevent a command to Robot A that would result in a collision as established by the connected OR controller.

Third party robots (e.g., robots of different origin) may interact in an OR. If a collision is expected, the system may notify the surgeon of the upcoming collision. Communication of the notification may be visual and/or non-visual. Non-visual communication may include sensory feedback (e.g., haptic and/or auditory) to create awareness of an approaching collision and/or system adjustment.

A system within the robotic human interface device may have a haptic motor that provides haptic feedback to warn the operator of the robotic system that the end effector or arm structure of a robotic arm is entering within a predefined radius of a person, obstacle, or other robotic arm system in the operating room. This radius may be considered the 'hazard zone.' As the distance between the potentially interfering object and the controlled robotic arm within the hazard zone decreases, the haptic and/or auditory feedback may increase proportionally to inform of the increasing risk of proximity/collision. A system within the robotic human interface device may include a system that physically resists manipulation of the human interface device by the operator if the end effector or arm of a robot enters a hazard zone. The system may resist control that would move the robot into a position of proximity or collision risk of another object. The resistance may increase as the distance between the potentially colliding object and the robotic arm decreases. This resistance may increase to the point where a user cannot move the controls into a position that would result in a collision of the controlled robotic arm and another object.

Collision prevention in a robotic system may be based on behavioral and user-feedback.

Short term procedure limitations during steps with less risk may be accepted to prioritize long term collision reduction during subsequent steps with higher surgical risk. If a system (e.g., always) selects the position or movement with the lowest risk of entanglement, the system may create many more risks of entanglements throughout the procedure.

The system may select an option in the beginning of a procedure that creates the risk of entanglement (e.g., but reduces the likelihood of them occurring further on the procedure). In a system that has more control of itself than simple extension/retraction, the system may use that control to help reduce overall entanglement throughout the procedure.

As the mechanical capabilities of the system grows, the system's algorithmic processing and complexity may grow (e.g., to match the scenarios it may accidentally create). Procedure steps (e.g., N steps) may be modeled in succession (e.g., with possible risk entanglements and comparison of overall possible future risk scores).

The device may determine, during a first step in the plurality of surgical procedure steps, a current arm position of the first robotic arm and a current arm position of the second robotic arm that are external to a patient. The device may determine, during a second step in the plurality of surgical procedure steps, the target end effector position of the first end effector. The first candidate motion and a second candidate motion of the first robotic arm may be identified based on the current arm positions of the first and second robotic arms and the plurality of steps of the surgical procedure.

One or more steps in the plurality of steps of the surgical procedure may involve use of a third robotic arm. The device may predict an effect, caused by the selected candidate motion of the first robotic arm, on a future motion of a third robotic arm. The control signal may indicate the effect.

Localized inefficiencies of movement may occur to create overall efficiencies in movement.

The system may recommend using handheld tool(s) to avoid collision. The range of motion/working space used may be reduced with human control. If a tool (e.g., endocutter, clip applier, etc.) is only used once, the system may recommend user intervention to optimize the number of tool exchanges and differing needs of working space for different tool types.

The system may detect a collision using sensors, force transducers, pressure sensors, electrical voltage from drive motors, sounds, and/or accelerometers. These devices may be mounted to the end of arm tool or smart module (e.g., clamped onto tool or built into the tool). These devices may be mounted to each end of a robot arm.

The data may be used in conjunction with a microprocessor to detect a stall, which may indicate a collision. Accelerometers (e.g., any two accelerometers) or other sensor data may be in synch with one another. For example, two acceleration/de-acceleration events (e.g., within microseconds) may indicate a stall or collision between the two arms.

The information may be used by the microprocessor to stop movement of the tools that collided. The information may be used to move the collided tools in a reverse motion to eliminate any collision forces. The information may be used to pause the effected tools. The information may be used to put affected tools in a limp mode where the forces are reduced so that no damage can be done to patient or equipment. The information may be used to send feedback to the surgeon or operating room staff. The information may be used to output alarms (e.g., audible, tactile, haptics, lights, etc.).

Stall detection may trigger corrective reaction. Microphones (e.g., attached to the end of arm tool, for example, clamped onto tool or built in, or to each end of a robot arm) may pick up the sound of collision between tools. The sound may be fed into a microprocessor for analysis. A library of collision sounds may be prerecorded such that the microprocessor can detect which tools collided. The recorded sounds from the microphones may be analyzed and compared with the prerecorded database of collision sounds. The information may be used by the microprocessor to stop movement of the tools that collided, move the collided tools in a reverse motion to eliminate any collision forces, pause the effected tools, put the affected tools in a limp mode where the forces are reduced so that no damage can be done to patient or equipment, send feedback to the surgeon or operating room staff, output alarms (e.g., audible, tactile, haptics, lights) and/or the like.

The likelihood of robotic-human collisions may be reduced. For example, non-contact-based sensing technologies may be used to identify the location of humans in close proximity to the robot. Sensing may be achieved using one the following: a light detection and ranging (LiDAR) scanner (e.g., that emits a laser to measure the distances of surrounding objects), ultrasonic sensors (e.g., that transmit an ultrasound wave that will bounce off an object or obstacle on its path and be detected by the receiver on the sensor, for example, to calculate the distance to objects in the area using time and the speed of sound), capacitance (e.g., electromagnetic fields that can detect human or foreign object presence as a capacitor, for example, similar to a Theremin instrument that can detect the relative location of a human hand between two antenna), a light curtain (e.g., a set of photoelectric sensors that can detect interruption of the signal between two points, a closed Wi-Fi network (e.g., between a Wi-Fi source (router/extender) and receivers (robot arms) to report signal round trip time).

Sensing technology may be agnostic to sensor positioning. Sensors may be embedded on one or more robotic arms and/or in the environment in one or more locations. Sensors may be embedded in a robotic arm in one or more locations.

A sensor or sensors may be placed in a centralized location or dispersed around the room to track the movement of the robotics arms in relation to other objects in the environment (e.g., humans).

A proximity system (e.g., with a controller independent of the robot) may be placed on each robot/robot arm (e.g., regardless of having one arm per robot or multiple arms per robot).

The system may differentiate robotic objects from foreign objects in the field. The robotic system may be aware of the position of each of the arms in the surgical field. The system may calculate whether the object measured in the field is a robotic arm or a foreign object (e.g., surgical assistant, patient, etc.).

The movement (e.g., change in distance) of objects in the field relative to known robotically controlled movement of the arms may be monitored. The movement may inform the system whether the object is part of the robotic system or an object in the field (e.g., surgical assistant, patient, etc.).

For example, if an object moves from one side of the field to the other (but the robotic arm is not moving that far), the system may conclude that the object is foreign body and likely a human.

The effect of sterile drapes (e.g., plastic bags) may be measured and accounted for in the system when measuring the distance between the robot and foreign objects. For example, the capacitance of the bag may be measured nulled from the calculation of whether a foreign object is in the field.

The robot may inform nearby users of movement (e.g., independent of sensors) so that the users can move out of the way of the robot. Movement advertising may be achieved through one or a combination of the following feedback methods: sound (e.g., beeping or other sound localized to the moving robotic arm), light (e.g., illuminating the joints in motion or showing direction of travel through light, for example, turn signals), and/or the like.

Robotic repositioning may be sufficiently slow to allow users time to react and move out of the way or press an emergency stop button on the arm to halt unwanted movement.

Manual override control in the OR (e.g., joint release button, OTP actuator, etc.) may allow surgical staff in the room to move arms or robots in a controlled manner. These techniques may help the system avoid human-robot collisions.

A collaborative multi-system decision may limit portions of device function to prevent inadvertent directed interactions. The collaboration may include an automated cooperative decision by at least two smart devices that reduce the operations of at least one of the two system to intercede before the two systems physically interact (e.g., based on the inputs from the user).

A simulation may be used as an input stream to complex HCP visualizations.

The system may have predictive capabilities to determine a potential collision from analyzing past macro surgical history and real-time pertinent data. The system may detect the potential collision one or more steps ahead of the collision and warn of the potential engagement.

The system may output visualization of upcoming collision. The system may ask the user for input.

Interactive smart systems may enable more understanding within the OR. If conflicts between the systems arise, a decision will be made. The system may convey the conflicts in a manner relevant to the surgeon so they can make a quick informed decision on how to proceed.

Tradeoff factors may be presented for surgeon decision making. Parameters to use for tradeoffs presented to surgeon may include risk (e.g., a risk percentage) of tools touching, an anticipated number of conflicts, downtime (e.g., for the patient post-operation), a number of clutch in/out, procedure duration, an interruption during a critical step, how to minimize the of conflicts (e.g., compared to instances in time), surgeon preference, procedure-specific factors, and/or the like.

The system may create surgeon profiles that defaults setup to selected preferences. The system may identify a first path as the preference (e.g., but does not stop procedure). The profile preferences may be changed by procedure steps. The system may allow the surgeon to select the recommended settings or customize the settings.

The system may inform the user of active decision-making (e.g., with one or more alternate options for the user to select). Parameters may be displayed to a surgeon during a procedure (e.g., when a decision or trade-off occurs based on the connected device set-up). Trade-offs may include time of procedure, movement of robotic arms to adjust positioning, risks of device collision, etc.

Trade-off and decision making may be minimal during the procedure. The display may not (e.g., should never) obstruct the center of the screen. A color indicator around the border of the screen may be used to highlight a decision to be made. A display message box may show a default choice (e.g., if the surgeon chooses to ignore it). The surgeon may have the ability to open the dialog box to view the trade-off scenario. The decision may be limited to two choices.

The space above the patient may not be of equal value (e.g., the space immediately above the trocar is in constant interchanging utilization, but the space above the head or leg is less useful). The system may change choices of robotic joint(s) to intentionally keep free the more important areas (e.g., where potential interaction could occur).

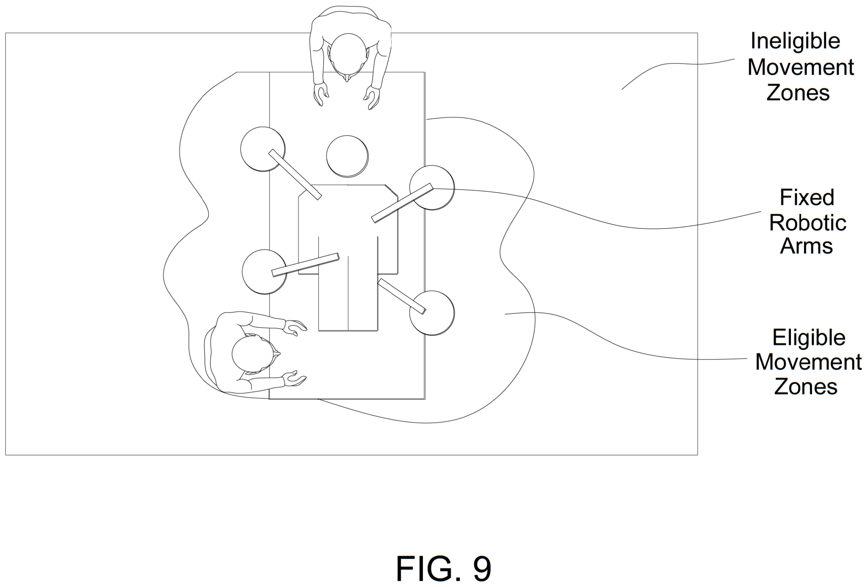

FIG. 9 illustrates an example display to a user of zones of robotic arm movements. The movement zones of individual consoles/arms may be used to predict ideal movement paths. The movement zones may be quantified by tool function, time, or risk associated with collisions. As shown in FIG. 9, there may be an ineligible movement zone. The ineligible movement zone may include areas outside of the reach of the robotic devices (e.g., because the bases holding the robotic arms are fixed), areas that are a threshold distance away from the patient or operating table, areas outside of a sterile barrier, and/or areas in which people or fixed devices will be during the procedure. The movement zones may include eligible movement zones. The eligible movement zones may include areas within reach of the robotic devices, areas within a threshold distance from the patient or operating table, and/or areas within the sterile barrier. The display movement zones may allow the surgeon to visualize limits to the robotic arms' movements.

A small fringe overlap of a movement space may be treated differently than an overlap in a critical section or zone of the instrument. A movement zone may be subdivided to present and map varying risks and importance within it. This subdivision may be performed (e.g., discretely, for example, with zones represented such as with boxes or discrete elements).

Subdivisions may be nested within one another to create context. Subdivisions may be computed (e.g., dynamically with a multitude of factors, resulting in more of a granular or analog subdivision).

Movement zones may be subdivided based on the relative locations corresponding to risk.

Not all movements or actions by a robotic system may make use of the space around them in the same manner. Some actuations or movements by a robotic system may not be significantly impacted if they are stopped or encounter interference.

Due to the temporal nature of actions within a surgery, the system may have a temporal understanding of functionality with movement zones.

The system may assign functionalities to movement zones or movement zone subdivisions. A movement path may be assigned a specific function, or a subdivision may be assigned a function.

Movement zones may be subdivided based on relative location corresponding to function. As the number of pieces of equipment increase, there may be no movement paths with zero risk. The most efficient movement path may incur some additional risk of interference compared to another movement path.

Overall risk of a conflict may be calculated in a variety of ways. For example, the system may calculate the overall overlap of collision space (e.g., the calculation of total area occupied (absolute or relative) of one or multiple movement paths). The system may calculate overlap or infringement of movement zones correlated to their risk. Pass/fail rating, weighted, or other criteria may be applied to the risk that is generated to determine if a path is eligible for movement. Movement paths may be deemed eligible based on overall risk of conflict.

The determination of eligible movement paths may depend on the severity of interference. Movement paths may represent the space that can be occupied.

Overlap or infringement of movement zones may be correlated to their assigned function.

If zones (e.g., representing similar and/or the same functions, or functions that may be co-dependent) are in conflict, it may change the way space is allocated within the room. Zones may have different functions, or work within their subsets of their respective movement spaces, without ever causing a conflict or collision to arise.

Space may be allocated (e.g., temporally) based on movement paths and tasks to be performed. The camera position may impact the simulation in 3D space. The cameras may triangulate robotic position in three dimensions. The hub and/or camera system may have to have stored parameter(s) related to the device(s) being tracked and/or capabilities of those device(s). Triangulation of the device position may be used to suggest device motions. For example, the system may use kinematics of the device(s) (e.g., robot arm(s)) and the patient to determine viable movement options. The kinematics may be derived (e.g., on the fly) using visualization. The range of motion (e.g., reach) and balance of the robot may be received from the robot or pre-determined.

A vision camera system may be placed over the operating room patient. One or more (e.g., at least 4) cameras may be used to see the robot arms and tools (e.g., including trocars). The cameras may gather 3D data by looking at the preprinted fiducials on the robotic arms and tools used by the robot.

CAD data of the arms and tools may be uploaded to further simplify the setup process. For each detected object, a path planning tool may calculate a full path of robot motion (e.g., to efficiently pick and move the tool while avoiding all collisions with the other arms in the system). The anti-collision operation of this system may use AI to adjust the arms and tools to not collide with other tools.

A layout may be presented to the surgeon. The layout may allow the surgeon to see the tools outside the patient on his/her control screen. For example, the display may be similar to a bird's eye view backup camera on a car where they could see an overhead view from above. If a tool collision were to happen, the bird's eye view may allow the surgeon to fix the positioning without addition help from personnel in the room.

The system may allow precise real-time 3D positioning of tools used around the patient. The data may be used for future enhancements (e.g., trocar setup, measurement tools, collision avoidance, big picture views for surgeon, etc.).

Indexing elements (e.g., fiducials) on the arms may enable a separate system to monitor the arms, (e.g., each of the segments of the arms). In some examples, electronic sensors may be used (e.g., rather than fiducials). The electronic sensors may emit a signal that is received by other sensors or a base station. For example, fiducials and/or electronic sensors may be placed at points 55318a-c (e.g., and/or other joints) in FIG. 5.

Fiducials may be printed or marked on the arm, device, etc. Fiducials may be a stick-on label, sterile tape, etc. that a vision system can calibrate and measure (prior to use in a setup step). Fiducials may be preprinted on device shaft and robotic arms. A clamp-on fiducial device may be added to tool shaft(s).

Patterns sprayed on a device may be used by the vision system to calibrate and measure the tool.

A linear encoder on a trocar may be used to determine axial position of the shaft with respect to trocar reference point. The shaft may be marked (e.g., with 2D bar code with ruler type markings).

A virtual trocar point may be spatially identified using fiducials and a 3D vision camera system. Robotic arm kinematics simulations may provide visualization of choices.

The system may determine alternative choices based on the procedure and the instrument capabilities and options. The system may determine a sharing relationship between two smart drives attempting to utilize the same space simultaneously (e.g., based on pre-determined aspects of the systems and their interaction). An aspect of the interaction may be the intensity or severity of the issue caused by the interaction, risk to the patient, or time criticality of one of the jobs, etc. that would be caused by the interaction. Instruments envelopes of operation may be determined based on risk or function of the instrument in causing collateral impacts to the patient or procedure, complexity of the path for the instrument to undo and take another path to avoid the other instrument, limitation to the instruments' functional capabilities (e.g., articulation angle), ease of display of the alternative options, and/or the like.

The system may determine boundary conditions of the joint movement simulation. The boundary conditions may be static. The system may identify safety limitations (e.g., danger areas for heated devices). The system may avoid critical structures when activating harmonic devices. The system may limit a joint from moving in a direction to ensure the device doesn't move toward a critical structure. If a device has a power cord, joint movement may be adapted to not interfere with cord movement.

One or more aspects may change between procedures. Some OR equipment positions may remain the same (e.g., the OR table, robotic console, ventilator, capital equipment tower, smoke evac, surgical energy, etc.). Some OR equipment positions may be semi-static (e.g., quasi set-in stone). For example, a Hugo robot arm base may be positioned and locked down.

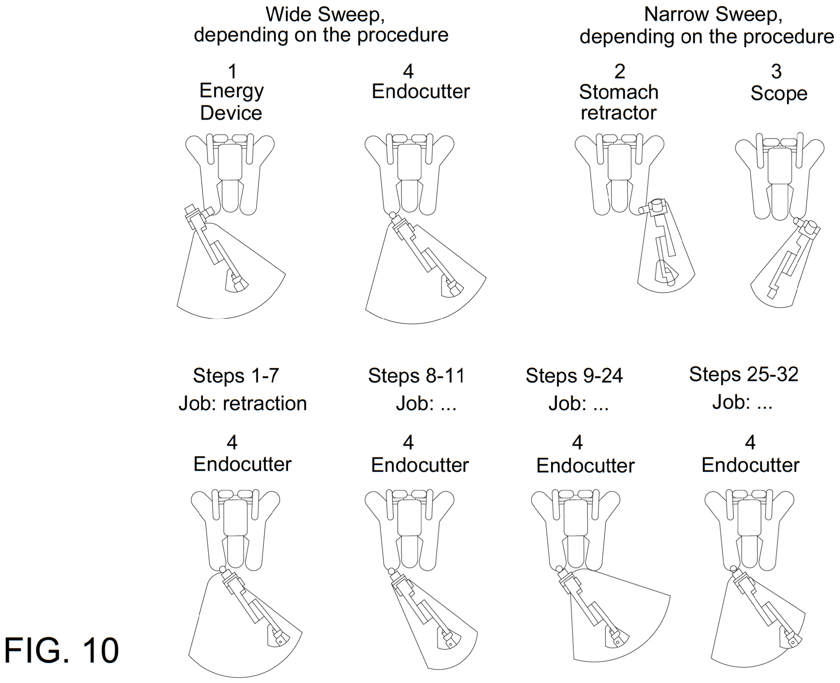

The procedure step may define when a zone is accessible (e.g., go or no-go). FIG. 10 illustrates example robotic arm movements based on the type of procedure being performed and/or a step of the procedure. For example, in certain procedures, arms controlling an energy device and an endocutter may be allowed to perform wide sweeps. For example, the energy device and endocutter may need to reach different areas of the body during the procedure, so they will be given the ability to move over large areas. This may limit the movement of other devices. Arms controlling some other devices (e.g., stomach retractor and scope in FIG. 10) may be allowed to only perform narrow sweeps. For example, the stomach retractor and scope may be moved little or not at all during the procedure (e.g., are mostly static), and won't need the ability to move over large areas. The limited movement may reduce the risk of these arms colliding with other arms (e.g., arms that will perform wide sweeps).

Boundaries may be static or dynamically change based on detected conditions. The system may determine if the plan was changed. The system may monitor for physiologic parameter shifts, user provided input, unexpected tool shifts on an arm, items changing during the procedure, and/or the like to determine changes in the plan.

The surgeon's view may change based on procedure type. For example, the view for mesentery mobilization differs from the view during anastomosis.

A scope may move between trocars (e.g., from trocar 2 to trocar 4). Within a (e.g., single) trocar, the endocutter may access various regions that depend on more proximal joint movement. The system may determine anticipated motion that will occur during the procedure. The distal tool depth and proximity to trocars may alter available space between arms.

The system may recommend a tool length (e.g., that allows a required tool depth). Motion may cause a change in arms/tools. For example, an HCP using a percutaneous retractor that cannot reach tissue may move to other side of the robot.

Unexpected circumstances (e.g., emergency physiologic needs) may occur. For example, an endocutter may misfire or arm motion may be different than initially planned (e.g., and might require human intervention).

Anticipated future steps may be used to determine the current arm movement. For example, at the end of a procedure with expected anastomosis, the surgeon may open space next to a natural orifice for access.

The system may determine whether a joint works better than others for the desired task. The system may determine whether this joint will cause a collision. The system may prioritize end effector position/use. The system may prioritize the number of arms/joints that will move (e.g., the system may avoid a collision by moving 4 arms or the system may create a potential interaction by only moving 1-2 arms). The system may deprioritize factors unrelated to the patient (e.g., stress on the tool).

Height of a tool base may be considered when determining tool position (e.g., impacts user access for exchanges). Tools that are exchanged or replaced may be fully retracted. The access point while the tool is in a fully retracted position may impact the speed of the tool exchange.

Access to distal end may be considered. For example, a scope may be manually wiped off when occluded.

The load on a retractor from the tissue may be translated through the robot arm. The system may determine joint placement to reduce (e.g., minimize) stress on robot arm joints and/or tools for long-term wear reduction. The first robot arm may include a plurality of joints configured to move the first robot arm. The device may select, from the plurality of joints, a joint of the first robotic arm to articulate to achieve the selected candidate motion.

As discussed with respect to FIG. 10, a scope arm may be a quasi-static boundary condition. The surgeon may prefer little to no movement of the scope to maintain a continuous field of view. Endocutter and retractor/energy device arms may have a greater range of motion. The endocutter and retractor/energy device may work cooperatively (e.g., communicating the desired space, and enacting boundary conditions for each other). For example, the retractor (2) may opt to minimize the angle of pitch at a joint (e.g., joint 6) to pull the head of the tool further away from endocutter (4) and energy device (1). Similarly, the energy device may opt to rotate one or more joints (e.g., joints 1, 2, and 3) to move the tool head furthest from the endocutter (4) (e.g., to give the endocutter more working space). The energy device (1) may use distal joints (e.g., roll, etc.) to perform its tasks.

An endocutter may be given maximum joint freedom to optimize the task at hand for critical firings. The endocutter may prioritize proximal joint movement to enable access angles. As shown in FIG. 10, the system may show a user multiple steps for one or more arms (e.g., to enable the user to indicate the most/least important regions of the body). For example, the endocutter may move in a wide sweep one or more times during steps 1-7 of the procedure. However, during steps 8-11, the endocutter may be relatively stationary. This information may allow the surgeon to better plan for when to move other arms. For example, there may be a lower risk of collision if other arms are moved during steps 8-11 than in steps 9-32 (e.g., when the endocutter moves in a wider area).

Each step in the plurality of steps of the surgical procedure may be associated with a surgical site internal to the patient, a second end effector is attached to a distal end of the second robotic arm. The device may identify a set of candidate motions, comprising the first candidate motion and the second candidate motion, based on the plurality of steps of the surgical procedure. Each candidate motion in the set of candidate motions may allow the first end effector and the second end effector to access the surgical site at a given step in the plurality of steps of the surgical procedure.

A static device may remain stationary for most of procedure. A dynamic device may move or be stationary based on the procedure step and/or outcome.

To visualize arm movement from one step to another, different shades within an arm may be used to indicate shifting. The visualization may allow the user to understand positions of multiple arms in different situations.

Other factors (e.g., beyond entanglement) may control the external kinematics of the joints. For example, historic data of collision/interaction, timeliness, sequencing, and/or the like may be used to determine kinematics.

The space used for different joints and/or arms may be kinematically sound. In some cases, if multiple movements are executed at the same time, the movements could cause collisions in the pathway to reach their destination. The system may consider the sequencing of movements during collision avoidance.

Movements may be performed in a specified order (e.g., order of operations, sequenced), or may be performed in parallel. The control signal may be configured to indicate the selected candidate motion of the first robotic arm. The control signal may be configured to indicate one or more of: the first candidate motion, the first number of interactions, the second candidate motion, the second number of interactions, a recommendation to move the first robotic arm according to the selected candidate motion, an order in which to perform the selected candidate motion and a motion of the second robotic arm, or a time at which to perform the selected candidate motion.

Space and time for starting positions, final positions, and/or transit paths may be temporally allocated to those functions and movements.

Entanglement and/or collisions may have degrees of conflict. There may be degrees of conflict that exceed the system's limits. The degrees of conflict may be within the limits that the surgeon and/or staff (e.g., the limits deemed critical). For certain movements or actions, the surgeon may want to push the system beyond what it normally would allow. Two arms or structures may push on one another and create entanglement concerns. That interaction may be advantageous in some way. The surgeon may override collisions or entanglement warnings.

If there is an upcoming instrument exchange, the instrument may be moved so that the tool driver can be manually switched. The system may identify eligible areas for an instrument exchange.

Instrument loading and dimensionality may be a factor for spatial isolation. If an end effector is changed on an autonomous system, an amount of space may be occupied to perform the swap. A very small end effector may not occupy much space and may only involve removing the end effector from the patient by a small amount. A larger end effector may involve fully extracting the current tool from the patient. The system may further retract away from the patient to allow sufficient space for a new tool to be installed into the system.

The system may have knowledge of a current instrument being used, an anticipated next instrument, confirmation of new instrument installation, and/or instrument-conveyed information.

The instrument may exchange information with the robotic system (e.g., without requiring manual information to be provided, such as information of length, type, etc. over an RFID or NFC communication method).

The user may (e.g., manually) input or confirm information regarding the instrument exchange and instrument installation.

Calibration and pre-run activities may be factors for spatial isolation. The instrument may be calibrated. The instrument may occupy a space during calibration. For example, an articulating harmonic device may allow arm movement, articulation of the end effector, and activation of the instrument in-air to confirm that all steps of the exchange have been properly performed (e.g., prior to insertion into the patient). These movements and activation may pose a potential risk to the HCPs and patient if they are in close proximity. The surgeon may ensure they are not co-located to the equipment.

The HCP location may be a factor for spatial isolation. For example, during an instrument exchange, the HCP may not stand in the same location occupied by another individual or piece of equipment. The system may anticipate where people and/or equipment are located, or use data streams (e.g., room cameras or triangulation of other equipment) to determine eligible locations for the instrument swap to occur.

Instrument swap complexity may be a factor for spatial isolation. A highly complicated swap of instruments may utilize additional space to perform the swap (e.g., as opposed to a low complexity swap of instruments).

For example, basic mechanical end effectors may be easily moved into locations that put a slight strain on the HCP (e.g., but allow for the surgery to be performed faster). The strain in this case may not be significant due to the ease of the instrument swap.

A more complicated, electro-mechanical interface with auxiliary connections may be more difficult to swap and may take longer for an HCP to perform. The mild strain that was acceptable for a faster swap may no longer be acceptable. In this case, the robotic system may move the instrument to a more accessible location.

Multi-step instrument exchanges may utilize multiple locations.

Complicated instrument assembly may involve moving the instrument to different locations for different stages of the instrument removal and assembly process (e.g., due to prior instrument removal, new instrument installation, auxiliary connections, electrical RF connections, new instrument calibration, position calibration, confirmation, such as scanning a barcode or button press, and/or the like).

The surgeon may deviate with the instrument swap from the planned instrument exchange (e.g., to use a different length, one of a different personal preference, or due to supply constraints).

The selected space may be (e.g., manually) modified. The new instrument may occupy more space than the system originally anticipated. The system may allow the user to enable new constraints or to manually move the end effector. The device may receive user preference information and a patient position associated with the surgical procedure. The device may determine a surgical constraint based on at least one of the user preference information or the patient position. The device may select the candidate motion of the first robotic arm, from the first candidate motion and the second candidate motion, based on the surgical constraint.

The user may manually override or modify the instrument confirmation. The new instrument may be different from the instrument that the system originally anticipated. This may result in modifications to the system's planned future movements. The system may send a confirmation of the impact to the surgical plan.

Leads and/or chords attached to the patient may be considered when determining device movement. For example, the patient may be attached to an IV, O2 supplementation, an EKG, a blood pressure cuff, energy wires, a monopolar ground pad, etc.

While the surgeon is viewing internal images at the robotic console, the system may display information related to external arm position.

If a procedure change occurs after the plan is determined, the system may provide options to the surgeon (e.g., options regarding how to proceed).

Although some aspects are described with respect to one or more robotic arms, a person of ordinary skill in the art will appreciate that these aspects may be used for any powered device (e.g., an articulable endocutter, etc.).

Example 1. A device comprising:
a processor configured to:
receive an indication of a plurality of steps of a surgical procedure associated with a patient, wherein one or more steps in the plurality of steps of the surgical procedure involve use of a first robotic arm having a first end effector attached and a second robotic arm;
identify a first candidate motion and a second candidate motion of the first robotic arm configured to place the first end effector in a target end effector position internal to the patient;
determine, for the first candidate motion, a first number of associated interactions in which the first robotic arm and the second robot arm co-occupy space external to the patient during the surgical procedure;
determine, for the second candidate motion, a second number of associated interactions in which the first robotic arm and the second robot arm co-occupy space external to the patient during the surgical procedure;
select a candidate motion of the first robotic arm, from the first candidate motion and the second candidate motion, based on the first number of interactions and the second number of interactions; and
generate a control signal based on the selected candidate motion of the first robotic arm.

Example 2. The device of example 1, wherein the processor is further configured to:
determine, during a first step in the plurality of surgical procedure steps, a current arm position of the first robotic arm and a current arm position of the second robotic arm that are external to a patient; and
determine, during a second step in the plurality of surgical procedure steps, the target end effector position of the first end effector, wherein the first candidate motion and a second candidate motion of the first robotic arm are identified based on the current arm positions of the first and second robotic arms and the plurality of steps of the surgical procedure.

Example 3. The device of example 1 or 2, wherein the control signal is configured to indicate the selected candidate motion of the first robotic arm.

Example 4. The device of any one of examples 1-3, wherein the control signal is configured to indicate one or more of:
the first candidate motion, the first number of interactions, the second candidate motion, the second number of interactions, a recommendation to move the first robotic arm according to the selected candidate motion, an order in which to perform the selected candidate motion and a motion of the second robotic arm, or a time at which to perform the selected candidate motion.

Example 5. The device of any one of examples 1-4, wherein each step in the plurality of steps of the surgical procedure is associated with a surgical site internal to the patient, a second end effector is attached to a distal end of the second robotic arm, and wherein the processor is further configured to:
identify a set of candidate motions, comprising the first candidate motion and the second candidate motion, based on the plurality of steps of the surgical procedure, wherein each candidate motion in the set of candidate motions allows the first end effector and the second end effector to access the surgical site at a given step in the plurality of steps of the surgical procedure.

Example 6. The device of any one of examples 1-5, wherein the processor being configured to select the candidate motion, from the first candidate motion and the second candidate motion, based on the first number of interactions and the second number of interactions comprises the processor being configured to:
on a condition that the first number of interactions is less than the second number of interactions, select the first candidate motion; and
on a condition that the first number of interactions is greater than the second number of interactions, select the second candidate motion.

Example 7. The device of any one of examples 1-6, wherein the target end effector position of the first end effector is a first position, and the processor is further configured to:
determine an updated current arm position of the first robotic arm, external to the patient, based on the first robotic arm moving according to the selected candidate motion;
determine a second target end effector position of the second end effector, during a third step in the plurality of surgical procedure steps, wherein the second target end effector position is internal to the patient;
determine, based on the updated current arm position of the first robotic arm, the current arm position of the second robotic arm, and the plurality of steps of the surgical procedure, a third candidate motion of the second robotic arm that will place the second end effector in the second target end effector position, wherein the third candidate motion of the second robotic arm is associated with a third number of interactions in which the first robotic arm and the second robot arm will co-occupy space during the surgical procedure;
determine, based on the updated current arm position of the first robotic arm, the current arm position of the second robotic arm, and the plurality of steps of the surgical procedure, a fourth candidate motion of the second robotic arm that will place the second end effector in the second target end effector position, wherein the fourth candidate motion of the second robotic arm is associated with a fourth number of interactions in which the first robotic arm and the second robot arm will co-occupy space during the surgical procedure;
select a candidate motion of the second robotic arm, from the third candidate motion and the fourth candidate motion, based on the third number of interactions and the fourth number of interactions; and
generate a control signal based on the selected candidate motion of the second robotic arm.

Example 8. The device of any one of examples 1-7, wherein one or more steps in the plurality of steps of the surgical procedure involve use of a third robotic arm, and the processor is further configured to predict an effect, caused by the selected candidate motion of the first robotic arm, on a future motion of a third robotic arm, wherein the control signal is further configured to indicate the effect.

Example 9. The device of any one of examples 1-8, wherein the processor is further configured to:
receive user preference information and a patient position associated with the surgical procedure; and
determine a surgical constraint based on at least one of the user preference information or the patient position, wherein the processor being configured to select the candidate motion of the first robotic arm, from the first candidate motion and the second candidate motion, is further based on the surgical constraint.

Example 10. The device of any one of examples 1-9, wherein the first robot arm comprises a plurality of joints configured to move the first robot arm, and the processor is further configured to select, from the plurality of joints, a joint of the first robotic arm to articulate to achieve the selected candidate motion.

Example 11. A method comprising:
receiving an indication of a plurality of steps of a surgical procedure associated with a patient, wherein one or more steps in the plurality of steps of the surgical procedure involve use of a first robotic arm having a first end effector attached and a second robotic arm;
identifying a first candidate motion and a second candidate motion of the first robotic arm configured to place the first end effector in a target end effector position internal to the patient;
determining, for the first candidate motion, a first number of associated interactions in which the first robotic arm and the second robot arm co-occupy space external to the patient during the surgical procedure;
determining, for the second candidate motion, a second number of associated interactions in which the first robotic arm and the second robot arm co-occupy space external to the patient during the surgical procedure;
selecting a candidate motion of the first robotic arm, from the first candidate motion and the second candidate motion, based on the first number of interactions and the second number of interactions; and
generating a control signal based on the selected candidate motion of the first robotic arm.

Example 12. The method of example 11, wherein the method further comprises:
determining, during a first step in the plurality of surgical procedure steps, a current arm position of the first robotic arm and a current arm position of the second robotic arm that are external to a patient; and
determining, during a second step in the plurality of surgical procedure steps, the target end effector position of the first end effector, wherein the first candidate motion and a second candidate motion of the first robotic arm are identified based on the current arm positions of the first and second robotic arms and the plurality of steps of the surgical procedure.

Example 13. The method of example 11 or 12, wherein the control signal is configured to indicate the selected candidate motion of the first robotic arm.

Example 14. The method of any one of examples 11-13, wherein the control signal is configured to indicate one or more of:
the first candidate motion, the first number of interactions, the second candidate motion, the second number of interactions, a recommendation to move the first robotic arm according to the selected candidate motion, an order in which to perform the selected candidate motion and a motion of the second robotic arm, or a time at which to perform the selected candidate motion.

Example 15. The method of any one of examples 11-14, wherein each step in the plurality of steps of the surgical procedure is associated with a surgical site internal to the patient, a second end effector is attached to a distal end of the second robotic arm, and wherein the method further comprises:
identifying a set of candidate motions, comprising the first candidate motion and the second candidate motion, based on the plurality of steps of the surgical procedure, wherein each candidate motion in the set of candidate motions allows the first end effector and the second end effector to access the surgical site at a given step in the plurality of steps of the surgical procedure.

Example 16. The method of any one of examples 11-15, wherein selecting the candidate motion, from the first candidate motion and the second candidate motion, based on the first number of interactions and the second number of interactions comprises:
on a condition that the first number of interactions is less than the second number of interactions, selecting the first candidate motion; and
on a condition that the first number of interactions is greater than the second number of interactions, selecting the second candidate motion.

Example 17. The method of any one of examples 11-16, wherein the target end effector position of the first end effector is a first position, and the method further comprises:
determining an updated current arm position of the first robotic arm, external to the patient, based on the first robotic arm moving according to the selected candidate motion;
determining a second target end effector position of the second end effector, during a third step in the plurality of surgical procedure steps, wherein the second target end effector position is internal to the patient;
determining, based on the updated current arm position of the first robotic arm, the current arm position of the second robotic arm, and the plurality of steps of the surgical procedure, a third candidate motion of the second robotic arm that will place the second end effector in the second target end effector position, wherein the third candidate motion of the second robotic arm is associated with a third number of interactions in which the first robotic arm and the second robot arm will co-occupy space during the surgical procedure;
determining, based on the updated current arm position of the first robotic arm, the current arm position of the second robotic arm, and the plurality of steps of the surgical procedure, a fourth candidate motion of the second robotic arm that will place the second end effector in the second target end effector position, wherein the fourth candidate motion of the second robotic arm is associated with a fourth number of interactions in which the first robotic arm and the second robot arm will co-occupy space during the surgical procedure;
selecting a candidate motion of the second robotic arm, from the third candidate motion and the fourth candidate motion, based on the third number of interactions and the fourth number of interactions; and
generating a control signal based on the selected candidate motion of the second robotic arm.

Example 18. The method of any one of examples 11-17, wherein one or more steps in the plurality of steps of the surgical procedure involve use of a third robotic arm, and the method further comprises predicting an effect, caused by the selected candidate motion of the first robotic arm, on a future motion of a third robotic arm, wherein the control signal is further configured to indicate the effect.

Example 19. The method of any one of examples 11-18, wherein the method further comprises:
receiving user preference information and a patient position associated with the surgical procedure; and
determining a surgical constraint based on at least one of the user preference information or the patient position, wherein selecting the candidate motion of the first robotic arm, from the first candidate motion and the second candidate motion, is further based on the surgical constraint.

Example 20. The method of any one of examples 11-19, wherein the first robot arm comprises a plurality of joints configured to move the first robot arm, and the method further comprises selecting, from the plurality of joints, a joint of the first robotic arm to articulate to achieve the selected candidate motion.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magnetooptical media, and optical media such as CD-ROM disks, and digital versatile disks (DVDs). A processor in association with software may be used to implement a radio frequency transceiver for use in a WTRU, UE, terminal, base station, RNC, or any host computer.

## Claims

1. A device comprising a processor configured to:
receive an indication of a plurality of steps of a surgical procedure associated with a patient, wherein one or more steps in the plurality of steps of the surgical procedure involve use of a first robotic arm having a first end effector attached and a second robotic arm;
identify a first candidate motion and a second candidate motion of the first robotic arm configured to place the first end effector in a target end effector position internal to the patient;
determine, for the first candidate motion, a first number of associated interactions in which the first robotic arm and the second robot arm co-occupy space external to the patient during the surgical procedure;
determine, for the second candidate motion, a second number of associated interactions in which the first robotic arm and the second robot arm co-occupy space external to the patient during the surgical procedure;
select a candidate motion of the first robotic arm, from the first candidate motion and the second candidate motion, based on the first number of interactions and the second number of interactions; and
generate a control signal based on the selected candidate motion of the first robotic arm.

2. The device of claim 1, wherein the processor is further configured to:
determine, during a first step in the plurality of surgical procedure steps, a current arm position of the first robotic arm and a current arm position of the second robotic arm that are external to a patient; and
determine, during a second step in the plurality of surgical procedure steps, the target end effector position of the first end effector, wherein the first candidate motion and a second candidate motion of the first robotic arm are identified based on the current arm positions of the first and second robotic arms and the plurality of steps of the surgical procedure.

3. The device of any one of the preceding claims, wherein each step in the plurality of steps of the surgical procedure is associated with a surgical site internal to the patient, a second end effector is attached to a distal end of the second robotic arm, and wherein the processor is further configured to:
identify a set of candidate motions, comprising the first candidate motion and the second candidate motion, based on the plurality of steps of the surgical procedure, wherein each candidate motion in the set of candidate motions allows the first end effector and the second end effector to access the surgical site at a given step in the plurality of steps of the surgical procedure.

4. The device of any one of the preceding claims, wherein the processor being configured to select the candidate motion, from the first candidate motion and the second candidate motion, based on the first number of interactions and the second number of interactions comprises the processor being configured to:
on a condition that the first number of interactions is less than the second number of interactions, select the first candidate motion; and
on a condition that the first number of interactions is greater than the second number of interactions, select the second candidate motion.

5. The device of any one of the preceding claims, wherein the target end effector position of the first end effector is a first position, and the processor is further configured to:
determine an updated current arm position of the first robotic arm, external to the patient, based on the first robotic arm moving according to the selected candidate motion;
determine a second target end effector position of the second end effector, during a third step in the plurality of surgical procedure steps, wherein the second target end effector position is internal to the patient;
determine, based on the updated current arm position of the first robotic arm, the current arm position of the second robotic arm, and the plurality of steps of the surgical procedure, a third candidate motion of the second robotic arm that will place the second end effector in the second target end effector position, wherein the third candidate motion of the second robotic arm is associated with a third number of interactions in which the first robotic arm and the second robot arm will co-occupy space during the surgical procedure;
determine, based on the updated current arm position of the first robotic arm, the current arm position of the second robotic arm, and the plurality of steps of the surgical procedure, a fourth candidate motion of the second robotic arm that will place the second end effector in the second target end effector position, wherein the fourth candidate motion of the second robotic arm is associated with a fourth number of interactions in which the first robotic arm and the second robot arm will co-occupy space during the surgical procedure;
select a candidate motion of the second robotic arm, from the third candidate motion and the fourth candidate motion, based on the third number of interactions and the fourth number of interactions; and
generate a control signal based on the selected candidate motion of the second robotic arm.

6. The device of any one of the preceding claims, wherein one or more steps in the plurality of steps of the surgical procedure involve use of a third robotic arm, and the processor is further configured to predict an effect, caused by the selected candidate motion of the first robotic arm, on a future motion of a third robotic arm, wherein the control signal is further configured to indicate the effect.

7. The device of any one of the preceding claims, wherein the processor is further configured to:
receive user preference information and a patient position associated with the surgical procedure; and
determine a surgical constraint based on at least one of the user preference information or the patient position, wherein the processor being configured to select the candidate motion of the first robotic arm, from the first candidate motion and the second candidate motion, is further based on the surgical constraint.

8. The device of any one of the preceding claims, wherein the first robot arm comprises a plurality of joints configured to move the first robot arm, and the processor is further configured to select, from the plurality of joints, a joint of the first robotic arm to articulate to achieve the selected candidate motion.

9. A method performed by a processor, the method comprising:
receiving an indication of a plurality of steps of a surgical procedure associated with a patient, wherein one or more steps in the plurality of steps of the surgical procedure involve use of a first robotic arm having a first end effector attached and a second robotic arm;
identifying a first candidate motion and a second candidate motion of the first robotic arm configured to place the first end effector in a target end effector position internal to the patient;
determining, for the first candidate motion, a first number of associated interactions in which the first robotic arm and the second robot arm co-occupy space external to the patient during the surgical procedure;
determining, for the second candidate motion, a second number of associated interactions in which the first robotic arm and the second robot arm co-occupy space external to the patient during the surgical procedure;
selecting a candidate motion of the first robotic arm, from the first candidate motion and the second candidate motion, based on the first number of interactions and the second number of interactions; and
generating a control signal based on the selected candidate motion of the first robotic arm.

10. The method of claim 9, wherein the method further comprises:
determining, during a first step in the plurality of surgical procedure steps, a current arm position of the first robotic arm and a current arm position of the second robotic arm that are external to a patient; and
determining, during a second step in the plurality of surgical procedure steps, the target end effector position of the first end effector, wherein the first candidate motion and a second candidate motion of the first robotic arm are identified based on the current arm positions of the first and second robotic arms and the plurality of steps of the surgical procedure.

11. The device of any one of claims 1 to 8 or the method of claim 9 or claim 10, wherein the control signal is configured to indicate the selected candidate motion of the first robotic arm, and/or wherein the control signal is configured to indicate one or more of:
the first candidate motion, the first number of interactions, the second candidate motion, the second number of interactions, a recommendation to move the first robotic arm according to the selected candidate motion, an order in which to perform the selected candidate motion and a motion of the second robotic arm, or a time at which to perform the selected candidate motion.

12. The method of any one of claims 9 to 11, wherein each step in the plurality of steps of the surgical procedure is associated with a surgical site internal to the patient, a second end effector is attached to a distal end of the second robotic arm, and wherein the method further comprises:
identifying a set of candidate motions, comprising the first candidate motion and the second candidate motion, based on the plurality of steps of the surgical procedure, wherein each candidate motion in the set of candidate motions allows the first end effector and the second end effector to access the surgical site at a given step in the plurality of steps of the surgical procedure.

13. The method of claim any one of claims 9 to 12, wherein selecting the candidate motion, from the first candidate motion and the second candidate motion, based on the first number of interactions and the second number of interactions comprises:
on a condition that the first number of interactions is less than the second number of interactions, selecting the first candidate motion; and
on a condition that the first number of interactions is greater than the second number of interactions, selecting the second candidate motion.

14. The method of any one of claims 9 to 13, wherein the target end effector position of the first end effector is a first position, and the method further comprises:
determining an updated current arm position of the first robotic arm, external to the patient, based on the first robotic arm moving according to the selected candidate motion;
determining a second target end effector position of the second end effector, during a third step in the plurality of surgical procedure steps, wherein the second target end effector position is internal to the patient;
determining, based on the updated current arm position of the first robotic arm, the current arm position of the second robotic arm, and the plurality of steps of the surgical procedure, a third candidate motion of the second robotic arm that will place the second end effector in the second target end effector position, wherein the third candidate motion of the second robotic arm is associated with a third number of interactions in which the first robotic arm and the second robot arm will co-occupy space during the surgical procedure;
determining, based on the updated current arm position of the first robotic arm, the current arm position of the second robotic arm, and the plurality of steps of the surgical procedure, a fourth candidate motion of the second robotic arm that will place the second end effector in the second target end effector position, wherein the fourth candidate motion of the second robotic arm is associated with a fourth number of interactions in which the first robotic arm and the second robot arm will co-occupy space during the surgical procedure;
selecting a candidate motion of the second robotic arm, from the third candidate motion and the fourth candidate motion, based on the third number of interactions and the fourth number of interactions; and
generating a control signal based on the selected candidate motion of the second robotic arm.

15. The method of any one of claims 9 to 14, wherein one or more steps in the plurality of steps of the surgical procedure involve use of a third robotic arm, and the method further comprises predicting an effect, caused by the selected candidate motion of the first robotic arm, on a future motion of a third robotic arm, wherein the control signal is further configured to indicate the effect.

16. The method of any one of claims 9 to 15, wherein the method further comprises:
receiving user preference information and a patient position associated with the surgical procedure; and
determining a surgical constraint based on at least one of the user preference information or the patient position, wherein selecting the candidate motion of the first robotic arm, from the first candidate motion and the second candidate motion, is further based on the surgical constraint.

17. The method of any one of claims 9 to 16, wherein the first robot arm comprises a plurality of joints configured to move the first robot arm, and the method further comprises selecting, from the plurality of joints, a joint of the first robotic arm to articulate to achieve the selected candidate motion.

18. A computer-readable medium comprising instructions which, when executed by a processor, cause the processor to carry out the method of any one of claims 9 to 17.
